# EUROPEAN PATENT SPECIFICATION

(11) **EP 3 520 701 B1**
(45) Date of publication and mention of the grant of the patent: **07.04.2021**
(21) Application number: 19155142.3
(22) Date of filing: 01.02.2019
(51) Int. Cl.: A61B 8/00

(54) **ULTRASONIC DIAGNOSTIC APPARATUS AND CONTROL METHOD THEREOF**
ULTRASCHALLDIAGNOSEVORRICHTUNG UND STEUERUNGSVERFAHREN DAFÜR
APPAREIL DE DIAGNOSTIC ULTRASONIQUE ET PROCÉDÉ DE COMMANDE ASSOCIÉ

(30) Priority: 06.02.2018 KR 20180014416
(43) Date of publication of application: 07.08.2019
(73) Proprietor: Samsung Medison Co., Ltd., Hongcheon-gun, Gangwon-do, 25108 (KR)
(72) Inventor: LEE, Su Myeong, Gyeonggi-do (KR); KIM, Jung Soo, Gyeonggi-do (KR); SHIN, Seong Chul, Gyeonggi-do (KR)
(74) Representative: Grootscholten, Johannes A.M.

(56) References cited:
- JP-A- 2010 194 259
- US-A1- 2009 187 104
- US-A1- 2011 172 535

## Description

### BACKGROUND

### 1. Field

Embodiments of the present disclosure relate to an ultrasonic diagnostic apparatus and a control method thereof, more particularly, to a technology configured to allow a user to more easily find a desired image among images captured by an ultrasound probe.

### 2. Description of Related Art.

An ultrasonic diagnostic apparatus refers to an apparatus configured to irradiate ultrasonic signals to a target part inside of an object and receive ultrasonic signals, which are reflected from the object (echo ultrasonic signals), thereby noninvasively acquiring images about soft tissue layer or blood vessels by using information thereon.

An ultrasonic diagnostic apparatus is relatively compact and inexpensive in comparison with another type of diagnostic imaging apparatus, e.g., X-ray device, Computerized Tomography Scanner (CT), Magnetic Resonance Image (MRI), diagnostic nuclear medical apparatus. In addition, the ultrasonic diagnostic apparatus is capable of obtaining an image about the inside of the object in real time, and the ultrasonic diagnostic apparatus is safe because there is no risk of radiation exposure. Therefore, the ultrasonic diagnostic apparatus is widely used in medical examination at cardiology, abdomen, urology, and maternity clinics.

Therefore, the ultrasonic diagnostic apparatus includes an ultrasound probe configured to transmit ultrasonic signals to an object and receive response signals reflected from the object so as to acquire an ultrasound image of the inside of the object.

The ultrasound probe includes a piezoelectric layer in which a piezoelectric material in the ultrasound probe vibrates and converts an electrical signal into an acoustic signal, a matching layer configured to allow ultrasonic waves, which is generated in the piezoelectric layer, to be effectively transmitted to an object by reducing a difference in acoustic impedance between the piezoelectric layer and the object, lens configured to focus ultrasonic waves, which move to the front side of the piezoelectric layer, to a particular point, a sound absorbing layer configured to prevent the ultrasonic waves from moving to the rear side of the piezoelectric layer or prevent the image distortion by reflecting the ultrasonic waves, and a connecting portion electrically connected to the sound absorbing layer and the piezoelectric material.

However, upon acquiring an internal image of a target object, the ultrasound probe is inserted into the inside the target object for capturing an image, and an angle of an array, which is provided in the front portion of the ultrasound probe, is rotated to acquire the ultrasound image since the ultrasound probe cannot move due to the spatial limitation within the target object.

Therefore, a user acquires a desired image by rotating the array angle of the ultrasound probe. According to the conventional method, there is an inconvenience in manually adjusting the angle upon acquiring the image of an angle desired by the user.

As an example of the above or other prior reference is made here to JP2010194259, which may be considered to disclose the preamble of the independent claims.

### SUMMARY

Therefore, it is an aspect of the present disclosure to provide an ultrasonic diagnostic apparatus capable of more easily providing an image of an angle desired by a user among images acquired by an ultrasound probe.

Additional aspects of the present disclosure will be set forth in part in the description which follows and, in part, will be obvious from the description, or may be learned by practice of the present disclosure.

In accordance with an aspect of the disclosure, an ultrasonic diagnostic apparatus includes an ultrasound probe provided with an array having at least one transducer; a display configured to display an image captured by the array; and a body provided with a controller configured to, when at least one image is selected by a user among a plurality of images captured according to a rotation angle of the array, control a rotation angle of the array to allow the selected image to be displayed on the display.

The controller may obtain an image in real time by rotating the array when the ultrasound probe is inserted into the inside of an object.

The controller may sequentially display images, which are captured according to the rotation angle of the array on the display.

The controller may display the plurality of images, which is captured according to the rotation angle of the array, and an image, which is selected by the user, together with each other, on the display.

The controller may display the plurality of images and the image selected by the user, to be distinguished from each other.

The controller may display the image, which is selected by the user, on the display by enlarging the image selected by the user.

The ultrasound probe may further include a first inputter configured to receive the rotation angle and the rotation range of the array and the number of images displayed on the display, from the user.

The body may further include a second inputter configured to receive the rotation angle and the rotation range of the array and the number of images displayed on the display, from the user.

The ultrasound probe may include one of a 3D ultrasound probe, a matrix ultrasound probe or a free hand ultrasound probe.

The ultrasound probe may include an insertion-type ultrasound probe.

In accordance with an aspect of the disclosure, a control method of an ultrasonic diagnostic apparatus comprising an ultrasound probe provided with an array having at least one transducer, and a body, the control method includes capturing a plurality of images according to a rotation angle of the array; displaying the plurality of images captured by the array, on a display; and when at least one image is selected by a user among the plurality of images, controlling the rotation angle of the array to allow the selected image to be displayed on the display.

The capture of the plurality of images may include obtaining an image in real time by rotating the array when the ultrasound probe is inserted into the inside of an object.

The displaying of the plurality of images on the display may include sequentially displaying images, which are captured according to the rotation angle of the array, on the display.

The displaying of the plurality of images on the display may include displaying the plurality of images, which is captured according to the rotation angle of the array, and an image, which is selected by the user, together with each other on the display.

The displaying of the plurality of images on the display may include displaying the plurality of images and the image selected by the user, to be distinguished from each other.

The displaying of the plurality of images on the display may include displaying the image, which is selected by the user, on the display by enlarging the image selected by the user.

The ultrasound probe may further include a first inputter configured to receive the rotation angle and the rotation range of the array and the number of images displayed on the display, from the user.

The body further may further include a second inputter configured to receive the rotation angle and the rotation range of the array and the number of images displayed on the display, from the user.

The ultrasound probe may include one of a 3D ultrasound probe, a matrix ultrasound probe or a free hand ultrasound probe.

The ultrasound probe may include an insertion-type ultrasound probe.

### BRIEF DESCRIPTION OF THE DRAWINGS

These and/or other aspects of the disclosure will become apparent and more readily appreciated from the following description of embodiments, taken in conjunction with the accompanying drawings of which:
FIG. 1 is a perspective view illustrating an appearance of an ultrasonic diagnostic apparatus according to an embodiment;
FIG. 2 is a view illustrating an appearance of an ultrasound probe according to an embodiment;
FIG. 3 is a view illustrating an internal view of the ultrasound probe according to an embodiment;
FIG. 4 is a view illustrating a state before the ultrasound probe according to an embodiment is inserted into the inside of an object;
FIG. 5 is a view illustrating that the ultrasound probe according to an embodiment acquires a plurality of image after being inserted into the inside of the object;
FIG. 6 is a block diagram illustrating a configuration of some component of ultrasonic diagnostic apparatus according to an embodiment;
FIG. 7 is a view illustrating that the ultrasound probe obtains an internal image of the object according an embodiment;
FIG. 8 is a view illustrating a screen on which the plurality of images obtained by the ultrasound probe is displayed on a display, according an embodiment;
FIG. 9 is a view illustrating a screen on which an image, which is selected by a user among the plurality of images, is displayed on the display, according an embodiment;
FIG. 10 is a view illustrating that the ultrasound probe obtains an internal image of the object according another embodiment;
FIG. 11 is a view illustrating a screen on which the plurality of images obtained by the ultrasound probe is displayed on a display, according another embodiment;
FIG. 12 is a view illustrating a screen on which an image, which is selected by a user among the plurality of images, is displayed on the display, according another embodiment; and
FIG. 13 is a flowchart illustrating a control method of the ultrasonic diagnostic apparatus according to an embodiment.

### DETAILED DESCRIPTION

Embodiments described in the present disclosure and configurations shown in the drawings are merely examples of the embodiments of the present disclosure, and may be modified in various different ways at the time of filing of the present application to replace the embodiments and drawings of the present disclosure.

Also, the terms used herein are used to describe the embodiments and are not intended to limit and / or restrict the present disclosure. The singular forms "a," "an" and "the" are intended to include the plural forms as well, unless the context clearly indicates otherwise.

In this present disclosure, the terms "including", "having", and the like are used to specify features, numbers, steps, operations, elements, components, or combinations thereof, but do not preclude the presence or addition of one or more of the features, elements, steps, operations, elements, components, or combinations thereof.

It will be understood that, although the terms first, second, third, etc., may be used herein to describe various elements, but elements are not limited by these terms. These terms are only used to distinguish one element from another element.

The description discloses the principles of the present disclosure and discloses embodiments of the present disclosure so that those skilled in the art will be able to practice the present disclosure while clarifying the scope of the present disclosure. The disclosed embodiments may be implemented in various forms.

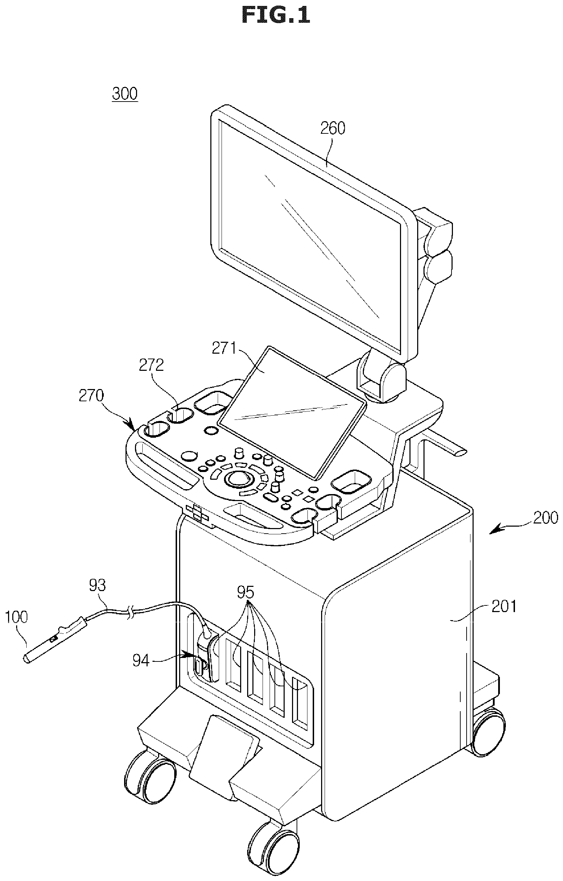

FIG. 1 is a perspective view illustrating an ultrasonic diagnostic apparatus 300 according to an embodiment having an ultrasound probe 100.

Referring to FIG. 1, the ultrasonic diagnostic apparatus 300 may include the ultrasound probe 100 transmitting and receiving ultrasonic waves to and from an object and a body 200 controlling an operation of the ultrasound probe 100 and generating an ultrasound image based on signals received from the ultrasound probe 100.

Particularly, the body 200 may control the overall operation of the ultrasonic diagnostic apparatus 300, and thus have various components for controlling the overall operation of the ultrasound probe 100 and the body 200. The body 200 and the ultrasound probe 100 may exchange data with each other using a cable 93 or a wireless communication module.

In addition, the ultrasound probe 100 and the body 200 may be connected to each other using the connection cable 93 as illustrated in FIG. 1. An electrical signal output from the ultrasound probe 100 may be transmitted to the body 200 through the connection cable 93. A control command generated in the body 200 may also be transmitted to the ultrasound probe 100 through the connection cable 93.

A connector 94 may be provided at one end of the connection cable 93 and the connector 94 may be coupled to or disconnected from a port 95 provided on an exterior 201 of the body 200. When the connector 94 is coupled to the port 95, the ultrasound probe 100 and the body 200 may communicate with each other.

In addition, one side of the body 200 may be provided with a probe holder 292 allowing the ultrasound probe 100 to hold thereon. The probe holder 292 may be provided as many as the number of the ultrasound probe 100, and the probe holder 292 may be mounted to or detached from the body 200. When a user does not use the ultrasound probe 100, the user can keep the ultrasound probe 100 by holding it on the probe holder 292.

In addition, through the ultrasound probe 100 and the wireless communication network, the body 200 may receive an electrical signal output from the ultrasound probe 100, and the body 200 may transmit an electrical signal generated by itself to the ultrasound probe 100. In this case, a wireless communication module including an antenna and a wireless communication chip may be installed in each of the ultrasound probe 100 and the body 200.

The wireless communication module may be a short range wireless communication using as least one of Bluetooth, Bluetooth low energy, infrared data association (IrDA), Wi-Fi, Wi-Fi Direct, ultra-wideband (UWB), and Near Field Communication (NFC). Alternatively, the wireless communication module may be a wireless communication module supporting 3GPP series, 3GPP2 series, or IEEE series wireless communication network authenticated by the International Telecommunication Union (ITU).

By using a communicator, the body 200 may send and receive data with a hospital server or other medical device in a hospital, wherein the hospital server and other medical device are connected to the body 200 through Picture Archiving and Communication System (PACS). In addition, the body 200 may send and receive data according to Digital Imaging and Communications in Medicine (DICOM) standard, but is not limited thereto.

A display 280 may be coupled to the body 200 and output a variety of information received from the ultrasound probe 100 and the body 200.

Particularly, the display 280 may display an ultrasound image about a target part 98 inside of an object 99. The ultrasound image displayed on the display 280 may be a 2D ultrasound image, a 3D ultrasound image, or a Doppler image. In addition, various ultrasound images may be displayed according to an operation mode of the ultrasonic diagnostic apparatus 300.

According to an embodiment, the ultrasound image includes a color mode (C-mode) image and a Doppler mode (D-mode) image as well as an amplitude mode (A-mode) image, a brightness mode (B-mode) image, and a motion mode (M-mode).

As described below, an A-mode image refers to an ultrasound image indicating the size of ultrasonic signal corresponding to echo ultrasonic signal, a B-mode image refers to an ultrasound image indicating the size of the ultrasonic signal corresponding to the echo ultrasonic signal, as the brightness, and a M-mode image refers to an ultrasound image indicating a movement of an object with respect to time at a particular position. A D-mode image refers to an ultrasound image indicating a moving object, as a waveform using a Doppler effect, and a C-mode image refers to an ultrasound image indicating a moving object, as color spectrum form.

Therefore, the display 280 may be implemented by using well known ways such as Cathode Ray Tube (CRT), Liquid Crystal Display (LCD), Light Emitting Diode (LED), Plasma Display Panel (PDP), and Organic Light Emitting Diode (OLED).

An inputter 290 may be implemented in various manners such as a keyboard, a foot switch or a foot pedal.

For example, the keyboard may be implemented in hardware. The keyboard may include at least one of a switch, a key, a joystick and a trackball. Alternatively, the keyboard may be implemented in software such as a graphic user interface. In this case, the keyboard may be displayed on the display 280.

In addition, when the display 280 is implemented as a touch screen type, the display 280 may also perform functions as the inputter 290. That is, the body 200 may receive various commands from a user via at least one of the display 280 and the inputter 290. According to an embodiment, the display 280 illustrated in FIG. 1 may simultaneously perform a display function and an input function.

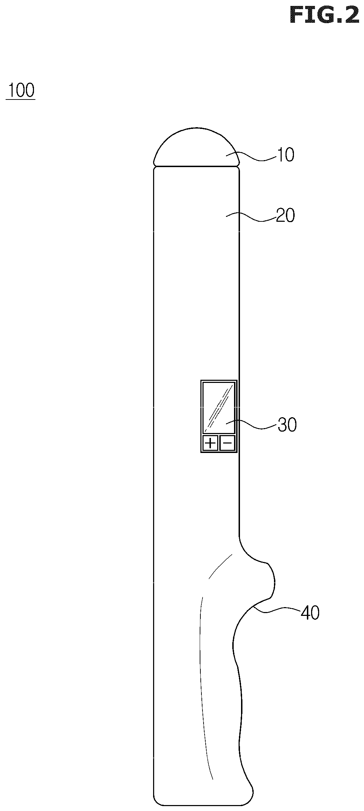

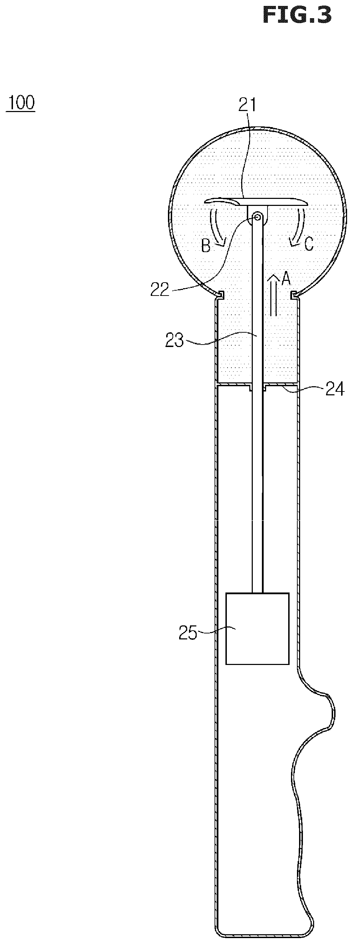

FIGS. 2 and 3 are views illustrating the outer and inner views of the ultrasound probe 100 according to an embodiment.

Referring to FIG. 2, according to an embodiment, the ultrasound probe 100 may include a head 10, a housing 20, a first inputter 30 and a handle 40.

The head 10 may be expanded by the pressure so as to form a space in which an array 21 is rotatable. Therefore, the head 10 may be formed of an elastic material that can be expanded by pressure.

The head 10 may be provided in the housing 20. Particularly, the housing 20 may have an opening, and the head 10 may be provided in the opening. At this time, the head 10 is brought into close contact with the opening so as to prevent leakage of the fluid and invasion of body fluids.

The first inputter 30 receiving information on a rotation range and a rotation angle of the array 21 from a user may be provided on one side of the housing 20. The handle 40 for a user of the ultrasonic diagnostic apparatus may be disposed on a lower end portion of the housing 20.

In addition, the ultrasound probe 100 illustrated in FIG. 2 may include one of a 3D ultrasound probe, a matrix ultrasound probe or a free hand ultrasound probe, or alternatively include an insertion-type ultrasound probe

Referring to FIG. 3, in the ultrasound probe 100, an inner array 21, a rotator 22, a support member 23, a partition wall 24 and a driver 25 may be provided.

The array 21 may include at least one transducer.

The array 21 may obtain an ultrasound image based on at least one transducer. The transducer may be implemented by any one of a magnetostrictive ultrasonic transducer using the magnetostrictive effect of a magnetic material, a piezoelectric ultrasonic transducer using the piezoelectric effect of a piezoelectric material, or a capacitive micromachined ultrasonic transducer (cMUT) that transmits and receives ultrasonic waves using vibration of several hundreds or thousands of micromachined thin films.

In addition, the transducer of the array 21 may be arranged in the form of a matrix, a linear, a convex, or a concave so as to obtain an ultrasound image. In addition, the array 21 may generate a 3D ultrasound image based on one or more transducers. Further, the array 21 may have a width greater than a diameter of the housing 20, and thus it may be possible to generate an ultrasound image having a wider angle or generate a sophisticated ultrasound image.

The rotator 22 may rotate the array 21. Particular, the rotator 22 may be provided on the rear surface of the array 21 so as to rotate the array 21 in the expanded head 10. An operation of the rotator 22 may be controlled by a controller 220.

By having a motor generating power and a gear coupled to the array 21 to transmit the power of the motor, the rotator 22 may rotate the array 21. At this time, the motor may be a stepping motor controlling a rotation angle.

The support member 23 may support the rotator 22 by being coupled to the rotator 22. A lower end of the support member 23 may be coupled to the driver 25.

The driver 25 may move the support member 24 forward or backward or rotate the support member 24. For example, the support member 24 and the driver 25 may be coupled to each other by a gear, and the driver 25 may move the support member 24 forward, backward, or rotate the support member 24 based on the power of the motor.

FIG. 3 illustrates that a single driver 25 moves the support member 24 forward or backward or rotates the support member 24. However, a driver 25 moving the support member 24 forward or backward may be provided separately from a driver 25 rotating the support member 24.

Meanwhile, a variety of devices required for driving the ultrasound probe 100 may be embedded in the support member 24, wherein the variety of devices may include a wire supplying electrical energy to the array 21 or the rotator 22, and a wire transmitting and receiving information to and from the array 21 or the rotator 22.

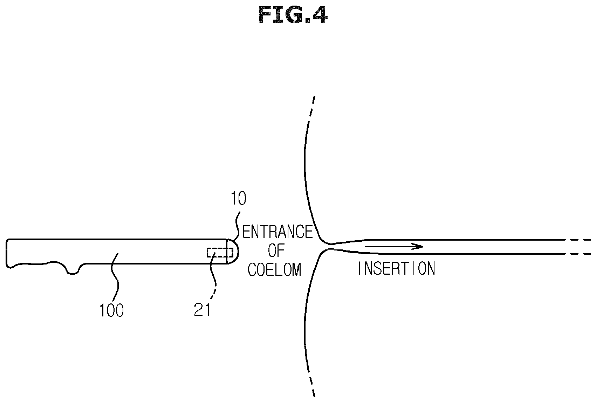

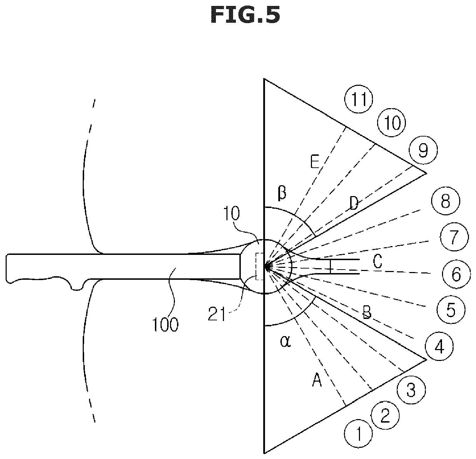

FIG. 4 is a view illustrating a state before the ultrasound probe 100 is inserted into the object, and FIG. 5 is a view illustrating a state after the ultrasound probe 100 is inserted into the object.

Referring to FIGS. 4 and 5, the ultrasound probe 100 according to an embodiment may be inserted into a subject, which is to be tested, and generate an ultrasound image of the subject while the array 21 is rotated in the subject. For example, the ultrasound probe 100 may be inserted into the coelom, anus, or vagina of the human body.

The ultrasound probe 100 may include the expandable head 10 and the housing 20. The array 21, which is illustrated in the dotted line of FIG. 4, may be provided in the ultrasound probe 100. The width of the array 21 may be greater than the diameter of the inside of the housing of the ultrasound probe 100.

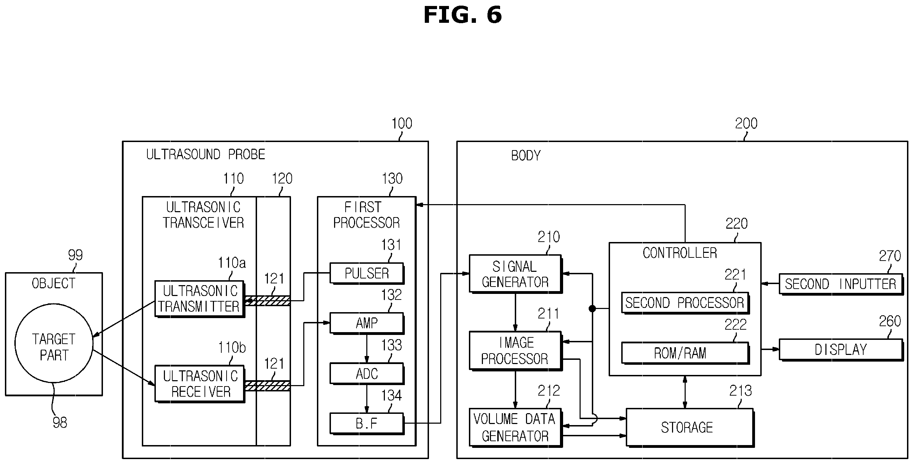

When the ultrasound probe 100 is inserted into the inside of the object, the ultrasound probe 100 may obtain at least one ultrasound image while the array 21 rotates, as illustrated in FIG. 6. The ultrasound probe 100 may obtain a more sophisticated ultrasound image or an ultrasound image having a wider field of view, by using the array 21 having the width greater than the diameter of the housing 20.

In addition, the array 21 may obtain an image of all directions while rotating within the object. For example, as illustrated in FIG. 5, the array 21 may obtain an internal image of the object while being positioned in from a direction ① to a direction ⑪. When obtaining an image in the direction ①, the array 21 may obtain an image within an angle range α and when obtaining an image in the direction ⑪, the array 21 may obtain an image within an angle range β.

FIG. 5 illustrates that the rotation range of the array 21 is from the direction ① to the direction ⑪, but is not limited thereto. According an embodiment, the ultrasound probe 100 may obtain a wider range image. An angle range that is obtained in a single direction, is not limited to the range α and β and thus the angle range may be set as various ranges.

FIG. 6 is a block diagram illustrating a configuration of some component of the ultrasonic diagnostic apparatus 300 according to an embodiment.

Referring to FIG. 6, the ultrasound probe 100 may include an ultrasonic transceiver 110 configured to generate or receive ultrasonic waves, and a first processor 130 electrically connected to the ultrasonic transceiver 110 and configured to control an operation of the ultrasonic transceiver 110 or configured to perform a signal processing using an electrical signal output from the ultrasonic transceiver 110.

The ultrasonic transceiver 110 may include an ultrasonic transducer that generates an ultrasonic wave or an electrical signal corresponding to the ultrasonic wave, may include an ultrasonic transmitter 110a and an ultrasonic receiver 110b.

The ultrasonic transmitter 110a may generate an ultrasonic wave having a frequency corresponding to a frequency of a pulse signal according to a pulse signal transmitted from the first processor 130 or the second processor 220. The generated ultrasonic wave may be irradiated to the target part 98 of the object 99.

The ultrasonic receiver 110b may receive an ultrasonic wave that is reflected by the target part 98 of the object 99 or that is generated on the target part 98 by laser, and convert the received signal into an ultrasonic signal. The ultrasonic receiver 110b may include a plurality of ultrasonic transducers, and since each of the ultrasonic transducer outputs an ultrasonic signal, the ultrasonic receiver 110b may output an ultrasonic signal of a plurality of channels.

In addition, the ultrasonic transceiver 110 may be installed on one surface of a sound absorber 120, and a first connection 121 corresponding to each of the ultrasonic transceiver 110 may be provided in the sound absorber 120.

According to an embodiment, the first connection 121 may be installed on the sound absorber 120 by passing through the sound absorber 120, and in this case, the first connection 121 may be installed in the sound absorber 120 by passing through from one surface to the other surface of the sound absorber 120.

The first processor 130 may generate or output an electrical signal to control the ultrasonic transceiver 110, or the first processor 130 may perform various types of signal processing by using the ultrasonic signal transmitted from the ultrasonic transceiver 110.

The electrical signal output from the first processor 130 may be transmitted to the ultrasonic transceiver 110 through the first connection 121 such as the ultrasonic transmitter 110a. The ultrasonic transmitter 110a may be driven by the received electrical signal.

The first processor 130 may include at least one of a pulser 131, an amplifier (AMP) 132, an analog-to-digital converter (ADC) 133, and a beam former 134.

The pulser 131 may generate a voltage of a predetermined frequency to drive the ultrasonic transceiver 110, and transmit the generated voltage to the ultrasonic transceiver 110. The ultrasonic transceiver 110 may generate ultrasonic waves by being vibrated according to an amplitude and frequency of the voltage output from the pulser 131.

The frequency and intensity of the ultrasonic waves generated by the ultrasonic transceiver 110 may be selected according to the amplitude and frequency of the voltage generated by the pulser 131. The voltage output from the pulser 131 may be applied to the ultrasonic transceiver 110 with a predetermined time difference so that the ultrasonic waves generated by the ultrasonic transceiver 110 are focused on the target part 98 or steered to a predetermined direction.

Depending on embodiments, the pulser 131 may be provided in a second processor 221. In this case, the first processor 130 may not include the pulser 131.

The amplifier 132 may amplify the ultrasonic signal output from the ultrasound receiver 110b of the ultrasonic transceiver 110. Depending on embodiments, the amplifier 132 may compensate for the strength difference between ultrasonic signals of a plurality of channels by amplifying ultrasonic signals of the plurality of channels, which are output from the plurality of ultrasonic transceivers 110, differently from each other.

When the amplified ultrasonic signal is an analog signal, the ADC 132 may convert the analogue signal into a digital signal. The ADC 132 may output a digital signal based on an ultrasonic signal corresponding to an analog signal, by performing sampling according to a predetermined sampling rate.

The beamformer (B.F) 134 may focus the ultrasonic signals input through the plurality of channels. The beam former 134 may generate a beamformed signal by focusing a signal transmitted from the ultrasonic transceiver 110, the amplifier 132, or the ADC 133. The beamformer 134 may perform electronic beam scanning, steering, focusing, apodizing, and aperture functions about a plurality of channels of signals.

When the ultrasound probe 100 is a wireless ultrasound probe, a battery (not shown) for supplying power to the ultrasound probe 100 may be additionally provided.

As illustrated in FIG. 6, the body 200 may include a signal processor 210, an image processor 211, a volume data generator 212, a storage 213, the display 280, a second inputter 290 and the controller 220.

The signal generator 210 may perform various signal processing on the beamformed signal. For example, the signal generator 210 may perform at least one of a filtering process, a detection process, and a compression process. The filtering process is a process for applying a filter to the beamformed signal to remove other signals than the signal of a certain bandwidth. The filtering process may include a harmonic imaging process removing fundamental frequency components and transmitting harmonic signals. The detection process is a process in which the voltage of an ultrasonic signal is converted from a radio frequency form into a video signal format. The compression process is a process for reducing the amplitude difference between ultrasonic signals. The signal generator 210 may be omitted, as needed.

The image processor 211 may convert the beamformed signal or the signal processed by the signal processor 210 into an ultrasound image in the form of a still image or moving image. As needed, the image processor 211 may perform a predetermined image processing on a still image or a moving image.

The image processor 211 may generate an ultrasound image through a scan conversion. The generated ultrasound image may include an A-mode image, B-mode image, M-mode, image Doppler mode image, or 3D image. The ultrasound image may include a Doppler image using the Doppler effect.

Further, the image processor 211 may correct the generated ultrasound image. For example, the image processor 211 may correct brightness, luminance, sharpness, contrast or color of all or a part of the ultrasound image so that a user clearly identifies a tissue on the ultrasound image. As needed, the image processor 211 may remove noise from the ultrasound image or perform pixel interpolation on the ultrasound image.

The image processor 211 may transmit the generated or corrected ultrasound image to the storage 213. In addition, the image processor 211 may transmit the generated or corrected ultrasound image to the volume data generator 212 so that the volume data generator 212 obtains ultrasound volume data.

The volume data generator 212 may obtain ultrasound volume data representing a three-dimensional volume using a two-dimensional ultrasound image generated or corrected by the image processor 211.

The above-described signal generator 210, the image processor 211, the volume data generator 212 may be implemented by a central processing unit or graphics processing unit. The central processing unit or graphics processing unit may be implemented using one or more semiconductor chips and associated components.

The storage 213 may store a variety of programs and data related to the function of the controller 220, the ultrasound image, and a variety of information on the ultrasound image. The storage 213 may be implemented by using a semiconductor storage, magnetic disk storage or magnetic tape storage device.

The display 280 may be coupled to the body 200 and output a variety of information received from the ultrasound probe 100 and the body 200.

Particularly, the display 280 may display an ultrasound image about the target part 98 inside of the object 99, and display a plurality of images obtained by the array 21 and an image, which is selected by a user among the plurality of images.

The second inputter 290 may receive various commands related to the operation of the ultrasound probe 100 and the body 200 from a user. Particularly, the first inputter 30 may receive information on the rotation range and the rotation angle of the array 21, from the user.

When the display 280 is implemented as a touch screen panel, the display 280 may simultaneously perform the function of the second inputter 290.

The controller 220 may control the overall operation of the ultrasonic diagnostic apparatus 300 according to a user's command or a predefined setting. For example, the controller 220 may generate a predetermined control command according to the frequency of the ultrasonic waves, which are to be irradiated, and then transmit the generated control command to the pulser 131 of the first processor 130. The pulser 131 may apply a voltage of a predetermined frequency to the ultrasound transceivers 110 according to the control command. Accordingly, the ultrasound transceivers 110 may generate an ultrasonic wave having the predetermined frequency so as to irradiate the ultrasonic wave to the target part 98 of the object 99.

The controller 220 may include the second processor 221 and the storage device 222 such as ROM or RAM assisting the operation of the second processor 221. The second processor 221 may be implemented by a central processing unit. The central processing unit may be implemented by one or more semiconductor chips and associated components.

When at least one image among the plurality of images, which is obtained according to the rotation angle of the array 21, is selected by a user, the controller 220 may control the rotation angle of the array 21 to allow the selected image to be displayed on the display 280.

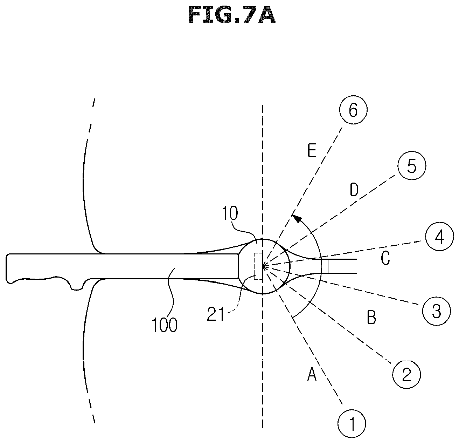

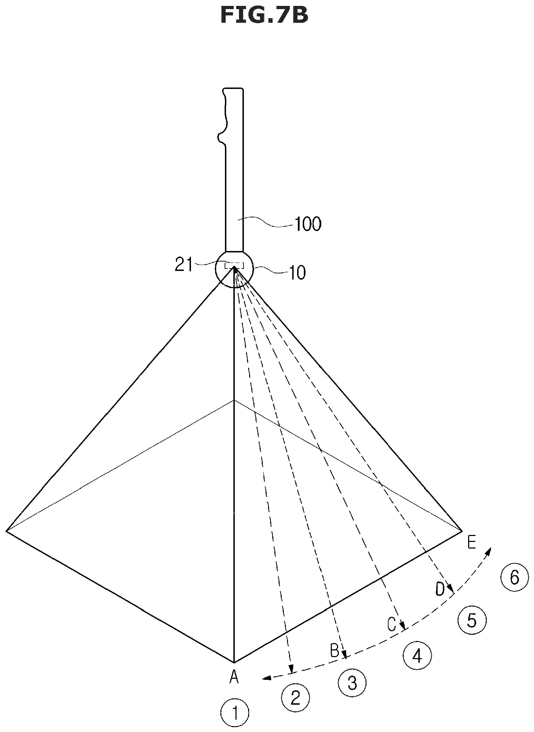

Particularly, when the ultrasound probe 100 is inserted into the inside of the object, the controller 220 may obtain an ultrasound image in various directions inside of the object by rotating the array 21, and display the obtained image on the display 280 . When a certain image is selected by a user among the plurality of images displayed on the display 280, the controller 220 may control the rotation angle and direction of the array 21 so that the selected certain image is displayed on the display 280. Hereinafter the controller 220 will be described in details with reference to FIGS. 7 to 11.

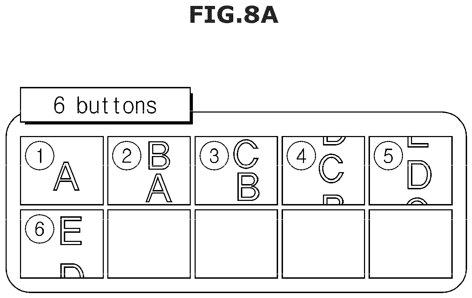

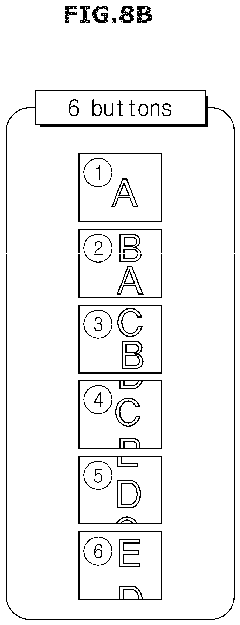

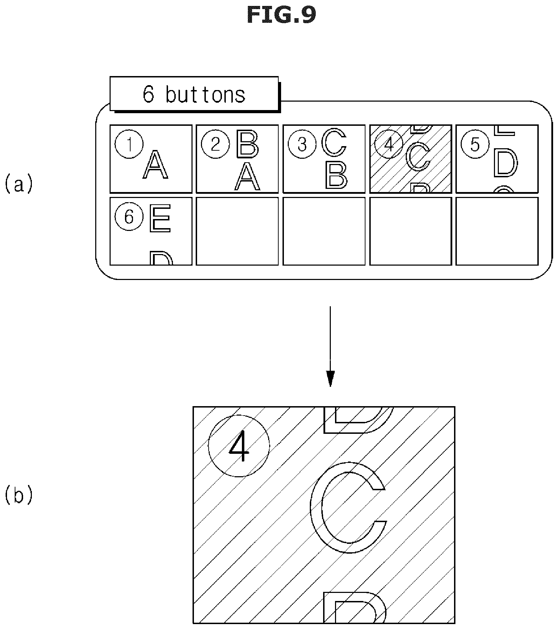

FIG. 7 is a view illustrating that the ultrasound probe 100 obtains an internal image of the object, according an embodiment. FIG. 7A illustrates that a 3D probe is inserted into the inside of the object and obtains images and FIG. 7B illustrates the obtained images as a 2D image. FIG. 8 is a view illustrating a screen on which the plurality of images obtained by the ultrasound probe 100 is displayed on the display 280, according an embodiment and FIG. 9 is a view illustrating a screen on which an image, which is selected by a user among the plurality of images, is displayed on the display 280, according an embodiment.

Referring to FIG. 7A, when the ultrasound probe 100 is inserted into the inside of the object, the controller 220 may obtain an image of the inside of the object by rotating the array 21 from the direction ① to the direction ⑥. When obtaining an image of the inside of the object by rotating the array 21 from the direction ① to the direction ⑥, it is possible to obtain images in an angle range X

For example, as illustrated in FIG. 7B, when the array 21 obtains an image by pointing the direction ①, the array 21 may obtain an image on which letter "A" is centered, and when the array 21 obtains an image by pointing the direction ④, the array 21 may obtain an image on which letter "C" is centered.

Therefore, when the array 21 obtains an image of the inside of the object by changing directions from the direction ① to the direction ⑥, an image obtained in each direction may be displayed on the display 280 as illustrated in FIG. 8.

A method of displaying an image on the display 280 may be displaying images in the lateral direction according to the obtaining order, as illustrated in FIG. 8A, or displaying image in the longitudinal direction according to the obtaining order, as illustrated in FIG. 8B.

Further, as illustrated in FIG. 8, the plurality of images displayed on the display 280 may be displayed such that images, which are obtained according to the rotation angle of the array 21, are updated in real time and then displayed or sequentially displayed. Alternatively, the plurality of images may be simultaneously displayed after the rotation of the array 21 is completed. A display method or a display order of displaying the plurality of images displayed on the display 280 may be variously set from a user through the first inputter 30 or the second inputter 290.

When a certain image is selected by a user among the plurality of images after displaying the plurality of images on the display 280, the controller 220 may control the rotation angle and direction of the array 21 so that the plurality of images and the selected image are displayed together with each other on the display 280.

That is, as illustrated in FIG. 9, when a user selects an image of the direction ④ to see an image on which letter "C" is centered, the controller 220 may rotate the array 21 to the direction ④ and thus the image of the direction ④ may be displayed on the display 280.

In addition, upon displaying the selected image, the controller 220 may enlarge the selected image and display the enlarged image so that a user takes a closer look at the image. In addition, on the screen on which the plurality of images is displayed, the selected image and the non-selected image may be distinguished from each other by using a shaded manner, as illustrated in FIG. 9A.

In addition, FIG. 9 illustrates that the screen displayed on the display 280 is changed into the selected image when a certain image is selected on the screen on which the plurality of images is displayed, but is not limited thereto. Therefore, the plurality of images and the selected image may be simultaneously displayed on the display 280. That is, the screen of FIG. 9A and the screen of FIG. 9B may be displayed on the display 280 as a single screen.

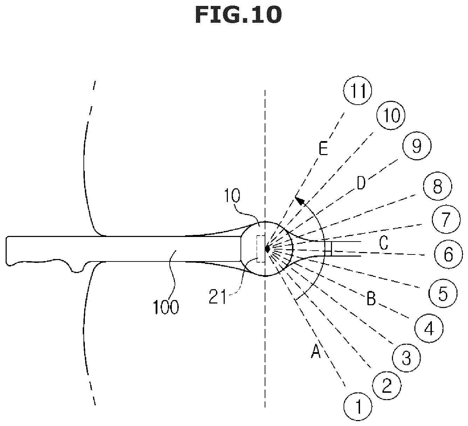

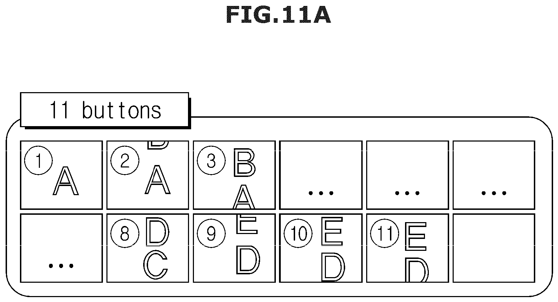

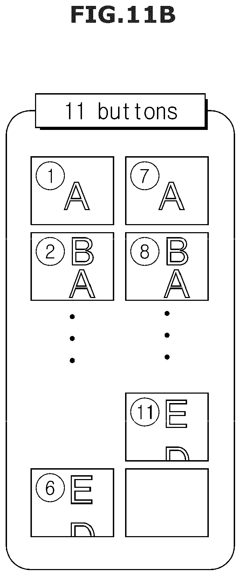

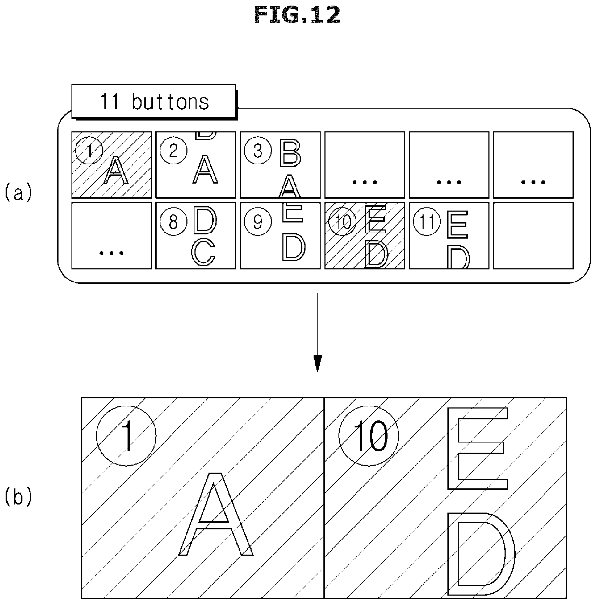

FIG. 10 is a view illustrating that an ultrasound probe 100 obtains an internal image of the object according another embodiment, FIG. 11 is a view illustrating a screen on which the plurality of images obtained by the ultrasound probe 100 is displayed on a display 280, according another embodiment, and FIG. 12 is a view illustrating a screen on which an image, which is selected by a user among the plurality of images, is displayed on the display 280, according another embodiment.

Referring to FIG. 10, when the ultrasound probe 100 is inserted into the inside of the object, a controller 220 may obtain an image of the inside of the object by rotating an array 21 from the direction ① to the direction ⑪. When obtaining an image of the inside of the object by rotating the array 21 from the direction ① to the direction ⑪, it is possible to obtain images in an angle range X

For example, when the array 21 obtains an image by pointing the direction ①, the array 21 may obtain an image on which letter "A" is centered, and when the array 21 obtains an image by pointing the direction the array 21 may obtain an image on which letter "D" is centered.

Therefore, when the array 21 obtains an image of the inside of the object by changing directions from the direction ① to the direction ⑪, an image obtained in each direction may be displayed on the display 280 as illustrated in FIG. 11.

A method of displaying an image on the display 280 may be displaying images in the lateral direction according to the obtaining order, as illustrated in FIG. 11A, or displaying image in the longitudinal direction according to the obtaining order, as illustrated in FIG. 11B.

Further, as illustrated in FIG. 11, the plurality of images displayed on the display 280 may be displayed such that images, which are obtained according to the rotation angle of the array 21, are updated in real time and then displayed or sequentially displayed. Alternatively, the plurality of images may be simultaneously displayed after the rotation of the array 21 is completed. A display method or a display order of displaying the plurality of images displayed on the display 280 may be variously set from a user through a first inputter 30 or a second inputter 290.

When a certain image is selected by a user among the plurality of images after displaying the plurality of images on the display 280, the controller 220 may control the rotation angle and direction of the array 21 so that the plurality of images and the selected image are displayed together on the display 280.

When a user selects an image of the direction ① to see an image on which letter "A" is centered, the controller 220 may rotate the array 21 to the direction ① and thus the image of the direction ① may be displayed on the display 280.

Further, as illustrated in FIG. 11, when a user selects an image of the direction ① to see an image on which letter "A" is centered and an image of the direction ⑩ to see an image on which letter "D" and "E" are centered, among the plurality of images, the controller 220 may rotate the array 21 to the direction ① and the direction ⑩ and thus the image of the direction ① and the direction ⑩ may be displayed on the display 280.

In addition, FIG. 11 illustrates that the screen displayed on the display 280 is changed into the selected image when a certain image is selected on the screen on which the plurality of images is displayed, but is not limited thereto. Therefore, the plurality of images and the selected image may be simultaneously displayed on the display 280. That is, the screen of FIG. 11A and the screen of FIG. 11B may be displayed on the display 280 as a single screen.

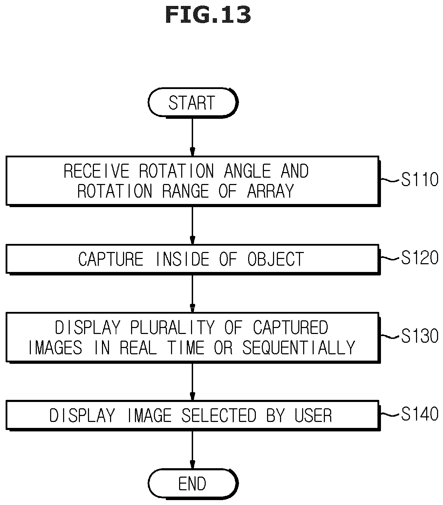

FIG. 13 is a flowchart illustrating a control method of the ultrasonic diagnostic apparatus 300 according to an embodiment.

Referring to FIG. 13, the ultrasonic diagnostic apparatus 300 may receive the rotation angle and the rotation range of the array 21 from a user (110).

The rotation range may represent an entire rotation range that can be captured by the array 21, and the rotation angel may represent a rotation range of the array 21 displayed on a single image. For example, when the rotation range is set as 180 degree, the array 21 may obtain an image having a range of total 180 degree, and when the rotation range is set as 30 degree, the array 21 may obtain six images (= 180/30). That is, an image having the total 30 degree may be obtained as a single image.

When the ultrasound probe 100 is inserted into the inside of the object, the array 21 may capture the inside of the object and acquire the image (120).

Particularly, the array 21 may capture the inside of the object based on the rotation range and rotation angle received from the user.

When the inside of the object is captured, the ultrasonic diagnostic apparatus 300 may display the captured plurality of images on the display 280 (130).

Particularly, the ultrasonic diagnostic apparatus 300 may display the plurality of images on the display 280 in real time or the ultrasonic diagnostic apparatus 300 may simultaneously display the plurality of images on the display 280 after capturing is completed.

Thereafter, the ultrasonic diagnostic apparatus 300 may receive a selection of at least one image from the user and display the selected image on the display 280 (140).

Particularly, the ultrasonic diagnostic apparatus 300 may control the rotation angle of the array 21 so that the image selected by the user is displayed on the display 280.

Hereinbefore the configuration and control method of the ultrasonic diagnostic apparatus 300 according to an embodiment have been described.

An ultrasonic diagnostic apparatus in the conventional manner has a difficulty in which a user should manually adjust an angel of the array of the ultrasound probe to obtain an image having an angle desired by the user.

However, as for the ultrasonic diagnostic apparatus 300 according to an embodiment, the angle of the array 21 may be automatically rotated when a user selects an image having an angle desired by the user among the plurality of images, and thus the user may more easily obtain the image having an angle desired by the user.

As is apparent from the above description, according to the proposed ultrasonic diagnostic apparatus, when a user selects an image having an angle desired by the user among the plurality of images obtained by the ultrasound probe, the rotation angle of the ultrasound probe is automatically controlled and thus it is possible to more easily provide the image having an angle desired by the user, to the user.

Hereinbefore a variety of embodiments is described, but is not limited thereto. A variety of embodiments which is implementable by those skilled in the art by correcting and modifying based on the above mentioned embodiment may correspond to the above mentioned embodiment. For example, when the above-mentioned techniques is executed in a different order from the above-mentioned method, and/or the above-mentioned components such as system, structure, device and circuit is coupled or combined in a manner different from the above-mentioned method or is replaced or substituted by other components or equivalents, the same or the similar result may be achieved. Therefore, other implementations, other embodiments are within the scope of the following claims.

## Claims

1. An ultrasonic diagnostic apparatus comprising:
an ultrasound probe provided with an array having at least one transducer;
a display configured to display an image captured by the array;
a body provided with a controller configured to, when at least one image is selected by a user among a plurality of images captured according to a rotation angle of the array, control a rotation angle of the array to allow the selected image to be displayed on the display,
**characterised in that**
the controller sequentially displays the plurality of images, which are captured according to the rotation angle of the array, and the image selected by the user, together with each other on the display.

2. The ultrasonic diagnostic apparatus of claim 1, wherein
the controller obtains an image in real time by rotating the array when the ultrasound probe is inserted into the inside of an object.

3. The ultrasonic diagnostic apparatus of claim 1, wherein
the controller displays the plurality of images and the image selected by the user, to be distinguished from each other.

4. The ultrasonic diagnostic apparatus of claim 1, wherein
the controller displays the image, which is selected by the user, on the display by enlarging the image selected by the user.

5. The ultrasonic diagnostic apparatus of claim 1, wherein
the ultrasound probe further comprises a first inputter configured to receive the rotation angle and the rotation range of the array and the number of images displayed on the display, from the user.

6. The ultrasonic diagnostic apparatus of claim 1, wherein
the body further comprises a second inputter configured to receive the rotation angle and the rotation range of the array and the number of images displayed on the display, from the user.

7. The ultrasonic diagnostic apparatus of claim 1, wherein
the ultrasound probe comprises one of a 3D ultrasound probe, a matrix ultrasound probe or a free hand ultrasound probe.

8. The ultrasonic diagnostic apparatus of claim 7, wherein
the ultrasound probe comprises an insertion-type ultrasound probe.

9. A control method of an ultrasonic diagnostic apparatus comprising an ultrasound probe provided with an array having at least one transducer, and a body, the control method comprising:
capturing a plurality of images according to a rotation angle of the array;
displaying the plurality of images captured according to the rotation angle of the array, on a display;
displaying the plurality of images, which is captured according to the rotation angle of the array, and an image, which is selected by the user, together with each other, on the display; and
when at least one image is selected by a user among the plurality of images, controlling the rotation angle of the array to allow the selected image to be displayed on the display.

10. The control method of claim 9, wherein
the capture of the plurality of images comprises
obtaining an image in real time by rotating the array when the ultrasound probe is inserted into the inside of an object.

11. The control method of claim 9, wherein
the displaying of the plurality of images on the display comprises.
displaying the plurality of images and the image selected by the user, to be distinguished from each other.

## Patentansprüche

1. Ultraschalldiagnosevorrichtung, die folgendes umfasst:
eine Ultraschallsonde, die mit einer Anordnung mit wenigstens einem Wandler versehen ist;
eine Anzeige, die dazu konfiguriert ist, ein von der Anordnung aufgenommenes Bild anzuzeigen;
einen Körper, der mit einer Steuerung versehen ist, die dazu konfiguriert ist, wenn wenigstens ein Bild aus einer Vielzahl von gemäß einem Drehwinkel der Anordnung aufgenommenen Bildern von einem Benutzer ausgewählt wird, einen Drehwinkel der Anordnung derart zu steuern, dass das ausgewählte Bild auf der Anzeige angezeigt werden kann,
**dadurch gekennzeichnet, dass**
die Steuerung nacheinander die Vielzahl von Bildern, die gemäß dem Drehwinkel der Anordnung aufgenommen wurden, und das von dem Benutzer ausgewählte Bild zusammen auf der Anzeige anzeigt.

2. Ultraschalldiagnosevorrichtung nach Anspruch 1, wobei
die Steuerung ein Bild in Echtzeit erhält, indem die Anordnung gedreht wird, wenn die Ultraschallsonde in das Innere eines Objekts eingeführt wird.

3. Ultraschalldiagnosevorrichtung nach Anspruch 1, wobei
die Steuerung die Vielzahl von Bildern und das von dem Benutzer ausgewählte Bild derart anzeigt, dass diese voneinander unterschieden werden können.

4. Ultraschalldiagnosevorrichtung nach Anspruch 1, wobei
die Steuerung das von dem Benutzer ausgewählte Bild auf der Anzeige anzeigt, indem das von dem Benutzer ausgewählte Bild vergrößert wird.

5. Ultraschalldiagnosevorrichtung nach Anspruch 1, wobei
die Ultraschallsonde weiterhin eine erste Eingabeeinrichtung umfasst, die dazu konfiguriert ist, den Drehwinkel und den Drehbereich der Anordnung sowie die Anzahl von auf der Anzeige angezeigten Bildern von dem Benutzer zu empfangen.

6. Ultraschalldiagnosevorrichtung nach Anspruch 1, wobei
der Körper weiterhin eine zweite Eingabeeinrichtung umfasst, die dazu konfiguriert ist, den Drehwinkel und den Drehbereich der Anordnung sowie die Anzahl von auf der Anzeige angezeigten Bildern von dem Benutzer zu empfangen.

7. Ultraschalldiagnosevorrichtung nach Anspruch 1, wobei
die Ultraschallsonde eine 3D-Ultraschallsonde, eine Matrix-Ultraschallsonde oder eine Freihand-Ultraschallsonde umfasst.

8. Ultraschalldiagnosevorrichtung nach Anspruch 7, wobei
die Ultraschallsonde eine Ultraschallsonde zum Einführen umfasst.

9. Steuerungsverfahren für eine Ultraschalldiagnosevorrichtung, die eine Ultraschallsonde, welche mit einer Anordnung mit wenigstens einem Wandler versehen ist, und einen Körper umfasst, wobei das Steuerungsverfahren Folgendes umfasst:
Aufnehmen einer Vielzahl von Bildern gemäß einem Drehwinkel der Anordnung;
Anzeigen der Vielzahl von Bildern, die gemäß einem Drehwinkel der Anordnung aufgenommen wurden, auf einer Anzeige;
Anzeigen der Vielzahl von Bildern, die gemäß dem Drehwinkel der Anordnung aufgenommen wurden, und eines von dem Benutzer ausgewählten Bildes zusammen auf der Anzeige; und
wenn wenigstens ein Bild aus einer Vielzahl von Bildern von einem Benutzer ausgewählt wird, Steuern eines Drehwinkels der Anordnung derart, dass das ausgewählte Bild auf der Anzeige angezeigt werden kann.

10. Steuerungsverfahren nach Anspruch 9, wobei
das Aufnehmen der Vielzahl von Bildern das Aufnehmen eines Bildes in Echtzeit umfasst, indem die Anordnung gedreht wird, wenn die Ultraschallsonde in das Innere eines Objekts eingeführt wird.

11. Steuerungsverfahren nach Anspruch 9, wobei
das Anzeigen der Vielzahl von Bildern auf der Anzeige das Anzeigen der Vielzahl von Bildern und des von dem Benutzer ausgewählten Bildes derart, dass diese voneinander unterschieden werden können, umfasst.

## Revendications

1. Appareil échographique de diagnostic comprenant :
une sonde échographique dotée d'un réseau possédant au moins un transducteur ;
un dispositif d'affichage conçu pour afficher une image réalisée par le réseau ;
un corps doté d'un organe de commande conçu, lorsqu'au moins une image est choisie par un utilisateur parmi une pluralité d'images réalisées selon un angle de rotation du réseau, pour commander l'angle de rotation du réseau afin de permettre l'affichage de l'image choisie sur le dispositif d'affichage ;
**caractérisé en ce que**
l'organe de commande affiche de manière séquentielle la pluralité d'images, réalisées selon l'angle de rotation du réseau, et l'image choisie par l'utilisateur, conjointement sur le dispositif d'affichage.

2. Appareil échographique de diagnostic selon la revendication 1, dans lequel
l'organe de commande obtient une image en temps réel grâce à une rotation du réseau lorsque la sonde échographique est introduite à l'intérieur d'un objet.

3. Appareil échographique de diagnostic selon la revendication 1, dans lequel
l'organe de commande affiche la pluralité d'images et l'image choisie par l'utilisateur, de façon à les distinguer les unes des autres.

4. Appareil échographique de diagnostic selon la revendication 1, dans lequel
l'organe de commande affiche l'image choisie par l'utilisateur sur le dispositif d'affichage en agrandissant l'image choisie par l'utilisateur.

5. Appareil échographique de diagnostic selon la revendication 1, dans lequel
la sonde échographique comprend en outre un premier organe d'entrée conçu pour recevoir l'angle de rotation et la plage de rotation du réseau ainsi que la quantité d'images affichées sur le dispositif d'affichage, en provenance de l'utilisateur.

6. Appareil échographique de diagnostic selon la revendication 1, dans lequel
le corps comprend en outre un second organe d'entrée conçu pour recevoir l'angle de rotation et la plage de rotation du réseau ainsi que la quantité d'images affichées sur le dispositif d'affichage, en provenance de l'utilisateur.

7. Appareil échographique de diagnostic selon la revendication 1, dans lequel
la sonde échographique comprend une sonde échographique 3D, une sonde échographique à matrice ou une sonde échographique à main.

8. Appareil échographique de diagnostic selon la revendication 7, dans lequel
la sonde échographique comprend une sonde échographique de type intra-cavitaire.

9. Procédé de commande d'un appareil échographique de diagnostic comprenant une sonde échographique dotée d'un réseau possédant au moins un transducteur, et un corps, le procédé de commande comprenant :
la réalisation d'une pluralité d'images selon un angle de rotation du réseau ;
l'affichage de la pluralité d'images réalisées selon l'angle de rotation du réseau, sur un dispositif d'affichage ;
l'affichage de la pluralité d'images, réalisées selon l'angle de rotation du réseau, et d'une image, choisie par l'utilisateur, conjointement, sur le dispositif d'affichage ; et
lorsqu'au moins une image est choisie par un utilisateur parmi la pluralité d'images, la commande de l'angle de rotation du réseau afin de permettre l'affichage de l'image choisie sur le dispositif d'affichage.

10. Procédé de commande selon la revendication 9, dans lequel
la réalisation de la pluralité d'images comprend l'obtention d'une image en temps réel par rotation du réseau lorsque la sonde échographique est introduite à l'intérieur d'un objet.

11. Procédé de commande selon la revendication 9, dans lequel
l'affichage de la pluralité d'images sur le dispositif d'affichage comprend l'affichage de la pluralité d'images et de l'image choisie par l'utilisateur, de façon à les distinguer les unes des autres.
